# EUROPÄISCHE PATENTSCHRIFT

(11) **EP 2 231 113 B1**
(45) Veröffentlichungstag und Bekanntmachung des Hinweises auf die Patenterteilung: **21.08.2013**
(21) Anmeldenummer: 08868661.3
(22) Anmeldetag: 22.12.2008
(51) Int. Cl.: A61K 9/00, A61K 31/00

(54) **PHARMAZEUTISCHE ZUSAMMENSETZUNG ZUR PROPHYLAXE UND/ODER SYMPTOMATISCHEN BEHANDLUNG VON ZYSTISCHER FIBROSE**
PHARMACEUTICAL COMPOUND FOR THE PROPHYLAXIS AND/OR SYMPTOMATIC TREATMENT OF CYSTIC FIBROSIS
COMPOSITION PHARMACEUTIQUE DESTINEE A LA PREVENTION ET/OU AU TRAITEMENT SYMPTOMATIQUE DE LA MUCOVISCIDOSE

(30) Priorität: 21.12.2007 DE 102007063535
(43) Veröffentlichungstag der Anmeldung: 29.09.2010
(73) Patentinhaber: Cycnad GmbH & Co. KG, 26409 Wittmund (DE)
(72) Erfinder: GULBINS, Erich, 45147 Essen (DE)
(74) Vertreter: Patentanwälte Ruff, Wilhelm, Beier, Dauster & Partner
(86) Internationale Anmeldenummer: PCT/EP2008/010996
(87) Internationale Veröffentlichungsnummer: WO 2009/083211

(56) Entgegenhaltungen:
- WO-A1-96/37198
- WO-A1-2005/025550
- WO-A2-2004/017949
- ALBOUZ S; VANIER M T; HAUW J J; LE SAUX F; BOUTRY J M; BAUMANN N: "Effect of tricyclic antidepressants on sphingomyelinase and other sphingolipid hydrolases in C6 cultured glioma cells" NEUROSCIENCE LETTERS, LIMERICK, IE, Bd. 36, Nr. 3, 29. April 1983 (1983-04-29) , Seiten 311-315, XP024370245 ISSN: 0304-3940 [gefunden am 1983-04-29]
- BAUMANN N; ET AL: "EFFECT OF TRICYCLIC ANTIDEPRESSANTS ON LYSOSOMAL SPHINGOMYELINASE ACTIVIT" NATO ADVANCED SCIENCE INSTITUTE SERIES A: LIFE SCIENCES, XX, XX, Bd. 150, 1. Januar 1988 (1988-01-01), Seiten 627-634, XP008023933 ISSN: 0258-1213

## Beschreibung

Die vorliegende Erfindung betrifft eine pharmazeutische Zusammensetzung zur Prophylaxe und/oder symptomatischen Behandlung von zystischer Fibrose und einen entsprechenden Inhalator.

Infektionen mit Pseudomonaden, insbesondere Pseudomonas aeruginosa, stellen ein großes klinisches Problem, insbesondere bei Patienten mit Immunsuppressionen, schweren Traumen und Verbrennungen, dar. So liegt die Letalität von beispielweise durch Pseudomonas aeruginosa verursachten Bakteriämien bei ca. 20 %. Liegt zusätzlich noch eine Pneumonie oder eine chirurgische Komplikation vor, kann die Mortalitätsrate bei diesem Typ von Bakteriämie sogar auf 30 bis 50 % steigen. Die klinische Bedeutung von Pseudomonas aeruginosa wird insbesondere dadurch verdeutlicht, dass dieser Pseudomonaden-Typ bei ca. 40 % aller Todesfälle von Patienten mit einer Beatmungs-induzierten Pneumonie eine entscheidende Rolle spielt.

Die wichtigste klinische Bedeutung hat Pseudomonas aeruginosa vor allem bei Patienten mit zystischer Fibrose (Mukoviszidose). Zystische Fibrose bzw. Mukoviszidose ist eine genetisch bedingte, autosomalrezessive angeborene Stoffwechselerkrankung. Sie beruht auf einer Mutation des Gens für den Chloridionenkanal CFTR (Cystic Fibrosis Transmembrane Conductance Regulator). Der genetische Defekt des CFTR-Proteins führt zu verschiedenen klinischen Problemen, insbesondere pulmonalen und gastrointestinalen Symptomen. Aus klinischer Sicht sind vor allem die pulmonalen Symptome problematisch. So kommt es im Laufe der zystischen Fibrose bei nahezu allen Patienten zu einer chronischen Pneumonie mit Pseudomonaden, insbesondere Pseudomonas aeruginosa, die den Hauptgrund für die Destruktion der Lunge und den vorzeitigen Tod dieser Patienten darstellt. Zystische Fibrose stellt zumindest in der Europäischen Union (EU) und den USA die häufigste autosomal rezessive Erbkrankheit dar. Mit einer Rate von einem erkrankten Kind auf 2.500 Geburten erkranken allein in der EU jährlich ca. 40.000 Kinder und junge Erwachsene an zystischer Fibrose.

In der WO 2004/017949 A2 wird die Verwendbarkeit diverser Substanzen zur Prophylaxe und/oder Therapie bestimmter Infektionserkrankungen beschrieben. Dort ist insbesondere die Verwendung von Amitriptylin und Imipramin zur systemischen Behandlung von viralen Infektionen beschrieben.

Des Weiteren sind aus der Fachliteratur Untersuchungen des Einflusses von Imipramin und Desipramin auf die Aktivität von Sphingomyelinase bekannt (S. Albouz et al.: "Effect of tricyclic antidepressants on sphingomyelinase and other sphingolipid hydrolases in C6 cultured glioma cells"; Neuroscience Letters, 36 (1983), 311-315 und N. Baumann et al.: "Effect of tricyclic antidepressants on lysosomal sphingomyelinase activity" NATO Advanced Science Institute Series A: Life Sciences, Bd. 150, 1. Januar 1988, 627-634).

Um die Prophylaxe und insbesondere die symptomatische Therapie von zystischer Fibrose zu verbessern und insbesondere aus dem Stand der Technik diesbezüglich bekannten Problemen Rechnung zu tragen, stellt sich die Erfindung die Aufgabe, eine pharmazeutische Zusammensetzung bereitzustellen, welche in besonderer Weise zur Prophylaxe bzw. symptomatischen Behandlung von zystischer Fibrose geeignet ist. Des Weiteren stellt sich die Erfindung die Aufgabe, eine entsprechende Applikationshilfe sowie ein entsprechendes Kit bereitzustellen.

Diese Aufgabe wird erfindungsgemäß gelöst durch eine pharmazeutische Zusammensetzung bzw. Medikament zur Prophylaxe und/oder symptomatischen Behandlung von zystischer Fibrose, insbesondere zur Prophylaxe und/oder Behandlung von bei zystischer Fibrose auftretenden Infektionen und/oder Infektionserkrankungen, umfassend zumindest ein Antidepressivum aus der Gruppe Trimipramin, Chlorprothixen, Fluoxetin, Amlodipin, Sertralin und kombinationen davon und vorzugsweise zumindest ein Dispersionsmittel und/oder zumindest ein pharmazeutisch verträgliches Trägermaterial.

Die Erfindung beruht unter anderem auf der überraschenden Feststellung, dass es in den Lungen-Epithelzellen von Mäusen, die kein Cftr (Cystic fibrosis transmembrane conductance regulator) in der Lunge exprimieren zu einer Akkumulation von Ceramid kommt. Im Falle von Cftr handelt es sich um das Maus-Äquivalent zu CFTR, weswegen Mäuse, die kein Cftr exprimieren, - sogenannte Cftr-defiziente Mäuse bzw. Cftr-Knockout-Mäuse, - geeignete Modelorganismen für zystische Fibrose darstellen. Mit anderen Worten ist bei Vorliegen von zystischer Fibrose die pulmonale Ceramid-Konzentration im Vergleich zu einem gesunden Organismus deutlich erhöht. Der Anstieg der Ceramid-Konzentration in den Epithelzellen der Lungen beruht dabei insbesondere auf einem pH-Wert-Anstieg (Alkalisierung) in intrazellulären Vesikeln. Durch den Anstieg des intravesikulären pH-Werts wird das Ceramid abbauende Enzym saure Ceramidase stärker gehemmt als das Ceramid freisetzende Enzym saure Sphingomyelinase. Dieses Ungleichgewicht bezüglich der Hemmung von saurer Ceramidase und saurer Sphingomyelinase verursacht eine Akkumulation oder Anreicherung von Ceramid in den intrazellulären Vesikeln von Lungen-Epithelzellen. Die Ceramid-Akkumulation führt schließlich zum Tod der Epithelzellen. Sterben die Zellen ab, gelangen Zellbestandteile, insbesondere DNA, in das Bronchiallumen. An diese Zellbestandteile können in der Lunge vorhandene Erreger, insbesondere Pseudomonaden, adhärieren. Dadurch wird eine mikrobielle Besiedelung bzw. Kolonisierung der Lunge gefördert, wodurch wiederum das Risiko steigt, dass sich bei den betroffenen Patienten die eingangs erwähnten pulmonalen Symptome von zystischer Fibrose manifestieren. Zudem führt Ceramid zu einer chronischen Entzündung in der Lunge Cftr-defizienter Mäuse.

Im Rahmen der Erfindung konnte anhand von Cftr-defizienten Mäusen bzw. Cftr-Knockout-Mäusen interessanterweise gezeigt werden, dass die erfindungsgemäße Zusammensetzung insbesondere bei intrapulmo-naler, vorzugsweise inhalativer, Verabreichung eine stärkere Hemmung der sauren Sphingomyelinase als der sauren Ceramidase bewirkt. Des Weiteren konnte gezeigt werden, dass die erfindungsgemäße Zusammensetzung zu einer weitgehenden Normalisierung der Ceramid-Konzentration in den Lungen-Epithelzellen der Versuchstiere führte. Auch eine signifikante Verringerung von entzündlichen Reaktionen der Lungen-Epithelzellen konnte nach Verabreichung der erfindungsgemäßen Zusammensetzung festgestellt werden. Mit besonderem Vorteil konnte außerdem gezeigt werden, dass sich nach Verabreichung der erfindungsgemäßen Zusammensetzung auch die Infektionsanfälligkeit der Versuchstiere gegenüber pulmonalen Erreger, insbesondere Pseudomonas aeruginosa, normalisierte.

In einer bevorzugten Ausführungsform liegt das Antidepressivum im Dispersionsmittel dispergiert vor. Das Dispersionsmittel ist vorzugsweise flüssig. Mit besonderem Vorteil stellt das Dispersionsmittel eine wässrige Flüssigkeit, insbesondere eine physiologische Flüssigkeit, dar. Erfindungsgemäß ist es besonders bevorzugt, wenn das Dispersionsmittel ein Lösungsmittel ist oder ein solches enthält. Bevorzugte Dispersionsmittel sind aus der Gruppe Wasser, Ethanol, Isopropanol und/oder Dimethylsulfoxyd (DMSO) ausgewählt. Beispielsweise kann es sich bei dem Dispersionsmittel um eine physiologische Elektrolyt-, Puffer- oder Salzlösung, beispielsweise Kochsalzlösung, handeln.

Die erfindungsgemäße Zusammensetzung liegt vorzugsweise in Form einer pharmazeutischen Formulierung vor. Die Formulierung selbst kann in gasförmigem, flüssigem, halbfestem oder festem Zustand erfolgen. Die Zusammensetzung liegt als Aerosol, Spray oder Dispersion vor. liegen. Eine flüssige, insbesondere wässrige, Formulierung ist besonders bevorzugt. Erfindungsgemäß kann die Zusammensetzung als flüssige Dispersion, insbesondere als Suspension oder Lösung, vorliegen. Vorzugsweise liegt die Zusammensetzung als wässrige Dispersion, besonders bevorzugt als wässrige Lösung, vor.

Die Zusammensetzung liegt in Form eines Inhalats oder Inhalationsmittels vor. Bei dem Inhalat bzw. Inhalationsmittel kann es sich dabei um ein Spray, insbesondere Nasen- oder Mundspray, oder um eine Tropflösung handeln.

Die Zusammensetzung weist einer weiteren geeigneten Ausführungsform Hilfs- oder Zusatzstoffe, beispielsweise Bindemittel, Weichmacher, Verdünnungsmittel, Gleitmittel, Antistatika, Antioxidantien, Adsorptionsmittel, Trennmittel, Dispergiermittel, Drageelack, Entschäumungsmittel, Filmbildner, Emulsionsmittel, Extender, Pigmente und/oder Füllstoffe auf. Falls in flüssiger Form vorliegend, kann die Zusammensetzung zum Beispiel Aroma-Verbesserungszusatzstoffe, Konservierungsmittel, Stabilisatoren und/oder Puffer aufweisen. Die Zusatzstoffe können anorganischer und/oder organischer Natur sein. Beispielsweise kann es sich bei den Zusatzstoffen um Fette, Fettalkohole, Fettsäuren, Wachse, Polysaccharide, Zucker, Proteine, Peptide, Aminosäuren, Vitamine, Spurenelemente, Salze, Säuren, Basen, Alkohole, Polymere und dergleichen handeln. Als Zucker kommen bevorzugt Monosaccharide, Disaccharide und/oder Zuckeralkohole in Betracht. Beispiele für geeignete Zucker können aus der Gruppe Glukose, Saccharose, Laktose, Threose, Galaktose, Mannitol und Sorbitol ausgewählt sein. Ein geeignetes Protein ist beispielsweise humanes Serumalbumin. Beispiele für geeignete Salze sind Natriumchlorid, Kaliumchlorid, Calciumchlorid und/oder Magnesiumchlorid. Weitere geeignete Hilfsstoffe sind beispielsweise aus der Gruppe Cetyl-Trimethyl-Ammoniumbromid, BDSA, Cholat, Deoxycholat, Polysorbat 20 und 80, Fusidinsäure und Lipide, insbesondere kationische Lipide, ausgewählt. Die erfindungsgemäße Zusammensetzung kann weiterhin einen oder mehrere Zusatzstoffe aus der Gruppe löslichkeitsverstärkende Additive, vorzugsweise Cyclodextrin, hydrophile Additive, vorzugsweise Mono oder Oligosaccharide, absorptionsfördernde Additive, vorzugsweise Cholat, Deoxycholat, Fusidinsäure oder Chitosan, kationische Tenside, vorzugsweise Cetyl-Trimethyl-Ammoniumbromid, viskositätserhöhende Additive, vorzugsweise Carboxymethylcellulose, Maltodextrin, Algininsäure, Hyaluronsäure oder Chondroitinsulfat, eine Freisetzungsmatrix, beispielsweise auf der Basis von Polyanhydrid oder Polyorthoester, ein Hydrogel, ein partikuläres, langsames Freisetzungs-Depot-System, vorzugsweise auf der Basis von Polylaktid-Co-Glykolid (PLG), ein Depot-Schaum, Stärke oder von Cellulose abgeleitete Systeme aufweisen. Weiterhin kann die erfindungsgemäße Zusammensetzung ein lipidbasiertes Trägermaterial, vorzugsweise in Form einer Emulsion und/oder auf Basis von Liposomen, Niosomen oder Micellen aufweisen. Des Weiteren kann die erfindungsgemäße Zusammensetzung auch Additive enthalten, welche "Bilayer-Anordnungen destabilisieren, vorzugsweise Phosphatidyl-Ethanolamin. Weitere mögliche Zusatzstoffe sind beispielsweise fusogene Additive, vorzugsweise Cholesterol-Hemisuccinat.

Weiterhin können erfindungsgemäß in Frage kommende pharmazeutisch verträgliche Zusatz- bzw. Hilfsstoffe flüchtig oder nicht flüchtig sein. Beispiele für geeignete Klassen solcher Hilfsstoffe sind gasförmige, im überkritischen Zustand befindliche, flüssige oder feste Lösungsmittel. Geeignete Hilfsstoffe sind daher insbesondere aus der Gruppe Wasser, Terpene, wie beispielsweise Menthol, Alkohole, wie beispielsweise Ethanol, Propylenglykol, Glycerin und andere ähnliche Alkohole, Dimithylformamid, Dimithylacetamid, Wachs, superkritisches Kohlendioxid, Trockeneis und Mischungen davon ausgewählt.

Weiterhin kann die erfindungsgemäße Zusammensetzung auch ein Treibgas, beispielsweise komprimierte Luft, Stickstoff, Hydrofluoralkane und dergleichen, enthalten. Im Prinzip können dabei alle möglichen Treibgase verwendet werden, welche inert sind gegenüber dem Antidepressivum und gegebenenfalls weiteren pharmazeutisch-aktiven Verbindungen, welche in der erfindungsgemäßen Zusammensetzung enthalten sind.

Wie bereits erwähnt, kann die erfindungsgemäße Zusammensetzung alternativ oder in Kombination zu einem Dispersionsmittel ein pharmazeutisch verträgliches Trägermaterial aufweisen. Geeignete Trägermaterialien sind in der Literatur ausführlich beschrieben (Remington's Pharmaceutical Sciences, 16. Ausgabe, 1980, Mack Publishing Co., herausgegeben von Oslo et al.) Das Trägermaterial kann in der erfindungsgemäßen Zusammensetzung in Form von Partikeln vorliegen, wobei die Partikel selbst eine nicht inhalierbare Partikelgröße aufweisen können. Beispielsweise kann das Trägermaterial Partikel mit einer Partikelgröße zwischen 10 und 500 µm, insbesondere 50 und 200 µm, aufweisen.

Liegt die erfindungsgemäße Zusammensetzung als Pulver, insbesondere pulverförmige Formulierung, vor, kommen grundsätzlich alle hierfür geeigneten Trägermaterialien in Betracht. Geeignete Beispiele sind Glukose, Laktose, Laktose-monohydrat, Saccharose, Trehalose, Zuckeralkohole, wie beispielsweise Mannitol oder Xylitol, Polylaktid und/oder Cyclodextrine handeln.

Als flüssige Trägermaterialien kommen bevorzugt Wasser, Pufferlösungen, Elektrolyt- bzw. Salzlösungen, wässrige Dextroselösungen und/oder Glykollösungen in Frage. Des Weiteren kann das Trägermaterial aus verschiedenen Ölen, insbesondere Petroleumölen, tierischen Ölen, pflanzlichen Ölen oder synthetischen Ölen, ausgewählt sein. So kann es sich bei den in Frage kommenden Ölen zum Beispiel um Erdnussöl, Sojabohnenöl, mineralische Öle, Sesamöl und dergleichen handeln. Geeignete pharmazeutische Hilfsstoffe sind aus der Gruppe Stärke, Cellulose, Talg, Glukose, Laktose, Saccharose, Gelatine, Malz, Reis, Mehl, Kalk, Silikagel, Magnesiumstearat, Natriumstearat, Glycerol-Monostearat, Natriumcholrid, getrocknete Magermilch, Glycerin, Propylenglykol, Wasser, Ethanol und andere ausgewählt.

Geeignete Puffer, insbesondere isotonische Puffer, können Wasser, Kochsalz, Phosphat, Citrat, Succinat, Acetat, Adipat, Benzoat, Laktat, Maleat, Phosphat, Tartrat, Borat, Tri-Hydroxymethyl-Aminomethan, Glycin, Histidin, und andere organischen Säuren bzw. deren Salze enthalten.

Weiterhin kann es erfindungsgemäß vorgesehen sein, dass die Zusammensetzung, insbesondere ein darin vorkommendes pharmazeutisch verträgliches Trägermaterial, einen oder mehrere Stabilisatoren, reduzierende Verbindungen, Antioxidantien und/oder komplexierende Verbindungen aufweist. Die Verwendung von Puffer, Stabilisatoren, reduzierenden Verbindungen, Antioxidantien und komplexierenden Verbindungen zur Herstellung von pharmazeutischen Zusammensetzungen ist dem Fachmann hinreichend bekannt (Wang et al., "Review of Excipients and pHs for Parenteral Products Used in the United States." J. Parent Drug Assn., 34(6):452-462(1980); Wang et al., "Parenteral Formulations of Proteins and Peptides: Stability and Stabilizers," J. Parent. Sci. and Tech., 42:S4-S26 (Supplement 1988); Lachman, et al., "Antioxidants and Chelating Agents as Stabilizers in Liquid Dosage Forms-Part 1, "Drug and Cosmetic Industry, 102(1): 36-38, 40 and 146-148 (1968); Akers, M.J., "Antioxidants in Pharmaceutical Products," J. Parent. Sci. and Tech.,I 36(5):222-228 (1988); and Methods in Enzymology, Vol. XXV, Colowick and Kaplan eds., "Reduction of Disulfide Bonds in Proteins with Dithiothreitol," by Konigsberg, pages 185-188).

Geeignete Salze sind beispielsweise Natriumchlorid, Dextrose, Mannitol, Saccharose, Trehalose und dergleichen. Weist die erfindungsgemäße Zusammensetzung ein flüssiges Trägermaterial auf, so ist es bevorzugt, dass das Trägermaterial einen pH-Wert zwischen ungefähr 4.5 und 8.5 aufweist. Weist die erfindungsgemäße Zusammensetzung dagegen ein pulverförmiges Trägermaterial auf, so ist es ebenfalls bevorzugt, wenn das Trägermaterial einen pH-Wert in einem physiologisch verträglichen und damit nicht toxischen Bereich aufweist.

Für eine exakte volumetrische Dosierung der erfindungsgemäßen Zusammensetzung kann eine Verdünnung des Antidepressivums mit einem pharmazeutisch inaktiven Hilfsstoff erforderlich sein, um dosierbare Mengeneinheiten zu erhalten, welche die Anforderungen an die Dosiergenauigkeit erfüllen. Sofern erforderlich, kann eine Verdünnung derart gewählt werden, dass die zu verabreichende Menge der erfindungsgemäßen Zusammensetzung, welche bevorzugt über einen Inhalator verabreicht wird, genau die gewünschte Dosis des Antidepressivums enthält.

Die Zusammensetzung ist für eine inhalative Verabreichung vorgesehen. Mit besonderem Vorteil konnte nachgewiesen werden, dass für eine intrapulmonale Verabreichung der Zusammensetzung geringere Mengen bzw. Dosen des Antidepressivums erforderlich sind als bei einer systemischen Verabreichung der Zusammensetzung, um einen zufriedenstellenden prophylaktischen und/oder therapeutischen Effekt zu erzielen. Dies gilt vor allem im Hinblick auf eine Verringerung der Aktivität der sauren Sphingomyelinase, eine Normalisierung der zellulären Ceramid-Konzentration, eine Verringerung von entzündlichen Reaktionen sowie eine Normalisierung der Infektionsanfälligkeit gegenüber Erregern. So kann bei einer intrapulmonalen Verabreichung das Antidepressivum in einer Dosierung verwendet werden, welche in etwa 1/10 bis 1/1000 der Dosierung entspricht, wie sie typischerweise bei einer systemischen, beispielsweise einer oralen oder intravenösen, Verabreichung verwendet wird. Mit anderen Worten ist es bevorzugt, wenn das Antidepressivum in der erfindungsgemäßen Zusammensetzung in einer Dosis vorliegt, die 1/10 bis 1/1000 einer systemisch, insbesondere oral oder intravenös, verabreichten Dosis des Antidepressivums entspricht. Bevorzugt weist die erfindungsgemäße Zusammensetzung eine Dosis des Antidepressivums zwischen 0,01 und 20 mg, insbesondere 0,1 und 10 mg, auf. Im Falle von Amytriptylin ist beispielsweise eine Dosis zwischen 0,1 und 10 mg zutreffend. Auf diese Weise lassen sich unerwünschte Nebenwirkungen von Antidepressiva, beispielsweise Müdigkeit und Erschlaffung, weitgehend vermeiden. Erfindungsgemäß kann es vorgesehen sein, dass das Antidepressivum bei einer inhalativen Verabreichung der Zusammensetzung in einer Dosis zwischen 0,01 und 20 mg/Tag, insbesondere 0,1 und 10 mg/Tag, zugeführt wird. Ein weiterer Vorteil besteht darin, dass bei einer intrapulmonalen Verabreichung der Zusammensetzung keine systemische Wirkung des Antidepressivums stattfindet. Mit anderen Worten entfaltet das Antidepressivum bei intrapulmonaler Verabreichung seine Wirkung ausschließlich im Lungengewebe und nicht auch in anderen Geweben.

Das Antidepressivum ist aus der Gruppe Trimipramin, Chlorprothixen, Fluoxetin, Amlodipin, Sertralin und Kombinationen davon ausgewählt. Erfindungsgemäß kann es insbesondere vorgesehen sein, dass das Antidepressivum in Form eines physiologisch verträglichen Salzes, insbesondere in Form eines Hydrochlorids, vorliegt.

In einer weiteren vorteilhaften Ausführungsform weist die pharmazeutische Zusammensetzung neben dem Antidepressivum zumindest einen weiteren therapeutischen Wirkstoff auf. Der Wirkstoff kann insbesondere ein Antikörper sein. Bei den Antikörpern handelt es sich gewöhnlich um Antikörper, welche gegen die saure Sphingomyelinase gerichtet sind. Wie bereits erwähnt, eignet sich die erfindungsgemäße Zusammensetzung insbesondere zur Prophylaxe bzw. symptomatischen Behandlung von Infektionen bzw. Infektionserkrankungen, welche bei zystischer Fibrose auftreten. Die Infektionen bzw. Infektionserkrankungen können erfindungsgemäß zumindest von einem Erreger aus der Gruppe Pseudomonas Aeruginosa, Burkholderia cepacia, Staphylococcus aureus und Haemophilus influenzae verursacht sein. Die erfindungsgemäße Zusammensetzung eignet sich zudem zur Prophylaxe und/oder Therapie einer chronischen Entzündung bei zystischer Fibrose.

Die Zusammensetzung liegt in einer vorteilhaften Ausführungsform sterilisiert vor. Zur Sterilisierung der Zusammensetzung kommen grundsätzlich alle dem Fachmann zur Sterilisierung von pharmazeutischen Zusammensetzungen, insbesondere pharmazeutischen Formulierungen, bekannten Sterilisierverfahren in Betracht.

In einer bevorzugten Ausführungsform ist die pharmazeutische Zusammensetzung einem Inhalator zugeordnet. Vorzugsweise ist die pharmazeutische Zusammensetzung in einem Inhalator enthalten. Bei dem Inhalator kann es sich beispielsweise um eine Sprüh- oder Sprayvorrichtung handeln, wie sie üblicherweise in Apotheken erhältlich ist. Der Inhalator selbst weist mit besonderem Vorteil ein Mundstück, einen Vernebler und eine Pumpe auf.

Die Zusammensetzung liegt in einer weiteren bevorzugten Ausführungsform als Aerosol vor. Vorzugsweise weist die Zusammensetzung Partikel, insbesondere Aerosol-Partikel, mit einem Durchmesser, insbesondere einem aerodynamischen Durchmesser, zwischen 0,1 und 12 µm, insbesondere 1 und 12 µm, bevorzugt 2 und 12 µm, auf. Unter einem aerodynamischen Durchmesser im Sinne der vorliegenden Erfindung soll der Durchmesser eines Partikels mit einer normierten Dichte, beispielsweise mit einer normierten Dichte von 1 g/cm³, verstanden werden, welcher in Luft unter normalen atmosphärischen Bedingungen die gleiche Sinkgeschwindigkeit hat wie der Partikel selbst.

Es wird auch eine aerosolierte bzw. versprühte pharmazeutische Zusammensetzung offenbart, umfassend Partikel mit einem Durchmesser, insbesondere aerodynamischen Durchmesser, zwischen 0,1 und 12 µm, wobei die Partikel zumindest ein pharmazeutisch verträgliches Trägermaterial und zumindest ein Antidepressivum aufweisen. Bezüglich weiterer Einzelheiten und Merkmale hierzu, insbesondere im Hinblick auf das Antidepressivum, das Trägermaterial sowie die Partikel, wird vollständig auf die bisherige Beschreibung Bezug genommen.

Des Weiteren wird von der vorliegenden Erfindung auch ein Inhalator, welcher die erfindungsgemäße Zusammensetzung enthält, erfasst. Bezüglich weiterer Merkmale und Einzelheiten wird ebenso auf die bisherige Beschreibung verwiesen.

Weiterhin wird ein Kit zur Prophylaxe und/oder symptomatischen Behandlung von zystischer Fibrose, insbesondere zur Prophylaxe und/oder Behandlung von bei zystischer Fibrose auftretenden Infektionen und/oder Infektionserkrankungen offenbart, wobei das Kit zumindest ein Antidepressivum und zumindest ein Dispersionsmittel umfasst. Das Kit kann als weitere Komponente beispielsweise eine Pufferlösung enthalten. Bevorzugt sind das Antidepressivum und das Dispersionsmittel gemeinsam in einem Behältnis enthalten. Das Behältnis kann insbesondere als Applikationshilfe für die Verabreichung des Antidepressivums und des Dispersionsmittels ausgebildet sein. Es kann weiterhin vorgesehen sein, dass das Antidepressivum und das Dispersionsmittel im Kit im Wesentlichen getrennt voneinander vorliegen. In dieser Ausführungsform kann das Antidepressivum in Pulver- oder Tablettenform vorliegen. Bezüglich weiterer Merkmale und Einzelheiten zu dem Kit, insbesondere zu dem Antidepressivum, Dispersionsmittel und der Applikationshilfe, wird auf die bisherige Beschreibung verwiesen.

Außerdem wird auch ein Kit offenbart, umfassend:
- eine Vorrichtung zum Aerosolieren bzw. Versprühen einer pharmazeutischen Zusammensetzung und eine Patrone bzw. Kartusche, welche mit der Vorrichtung verbunden ist bzw. an die Vorrichtung angeschlossen ist, und
- eine aerosolierbare bzw. versprühbare, inhalierbare pharmazeutische Zusammensetzung, umfassend zumindest ein Dispersionsmittel und/oder zumindest ein pharmazeutisch verträgliches Trägermaterial und zumindest ein Antidepressivum.

Die Vorrichtung zum Aerosolieren bzw. Versprühen der Zusammensetzung kann derart ausgebildet sein, dass eine Überdosierung vermieden wird. Dies kann beispielsweise durch eine elektronische Überwachung der zu verabreichenden Dosiermengen geschehen. Wird die Anzahl der vorgesehenen Dosen erreicht, können geeignete Abschalt- oder Sperr- bzw. Verriegelungselemente aktiviert werden, wodurch die Verabreichung der erfindungsgemäßen Zusammensetzung gestoppt oder zumindest für eine bestimmte Zeit ausgesetzt wird. Außerdem kann die Vorrichtung zum Aerosolieren der Zusammensetzung programmierbar sein, beispielsweise um die Häufigkeit der Verabreichung zu bestimmen. Darüber hinaus kann die Vorrichtung, sofern gewünscht, eine Vielzahl von Komponenten aufweisen, welche die Freisetzung der erfindungsgemäßen Zusammensetzung ermöglichen. Beispielsweise kann die Vorrichtung Komponenten aufweisen, welche es erlauben, das Timing von Versprühen relativ zur Inhalation zu kontrollieren. Weiterhin kann die Vorrichtung auch Komponenten enthalten, welche den Patienten eine Rückmeldung über die Rate und/oder das Volumen der Inhalation liefern. Weiterhin können Komponenten vorgesehen sein, welche einer übermäßigen Verabreichung vorbeugen, einen Gebrauch durch nicht befugte Personen verhindern und/oder den Dosierungsverlauf aufzeichnen. Des Weiteren kann die Vorrichtung auch derart ausgebildet sein, dass sie einen Patienten dazu veranlasst, lediglich eine bestimmte Dosierung für einen bestimmten Zeitraum zu verwenden, und danach dem Patienten anzeigt, dass dieser eine andere Dosierungsform verwenden soll. Hierfür kann die Vorrichtung derart programmiert sein, dass der Patient beispielsweise mit einer relativ hohen Dosis beginnt, welche relativ schnell verabreicht werden kann, und anschließend die Vorrichtung nur dann aktiviert wird, wenn eine zweite, geringere Dosis gewählt wird. Zudem kann die Vorrichtung patienten- oder arztspezifisch programmierbar sein. Die Vorrichtung kann auch derart programmierbar sein, dass sie größere oder geringere Mengen der pharmazeutischen Zusammensetzung freisetzt und insbesondere ein Aerosol in unterschiedlichen Geschwindigkeiten bildet. Obwohl an sich jede Vorrichtung verwendet werden kann, welche die erforderlichen Mengen der erfindungsgemäßen Zusammensetzung zu den Lungen eines Patienten befördert, ist eine Vorrichtung mit der Bezeichnung Aradigm AerxEssence^{®} bevorzugt. Eine geeignete Vorrichtung, bei welcher es sich vorzugsweise um einen Inhalator, insbesondere elektronischen Inhalator, handelt, ist in der US 5,718,222 mit dem Titel "Disposable package for use in aerosolized delivery of drugs" beschrieben. So kann die erfindungsgemäße Zusammensetzung mit Hilfe des in der US 5,718,222 beschriebenen Inhalators verabreicht werden.

Die Vorrichtung kann eine oder mehrere Austragsöffnungen für die zu aerosolierende bzw. zerstäubende Zusammensetzung aufweisen. Bei der bzw. den Austragsöffnungen kann es sich insbesondere um Öffnungen oder Poren einer porösen Membran handeln, durch welche die Zusammensetzung im Zuge ihrer Verabreichung gepresst wird, um ein Aerosol zu erzeugen. Die Öffnungen bzw. Poren können dabei alle eine einheitliche Größe und insbesondere einheitliche Entfernungen zueinander aufweisen. Es ist jedoch ebenso möglich, dass die Öffnungen bzw. Poren unterschiedliche Größen besitzen und insbesondere unregelmäßig auf der Membran angeordnet sind. Wird die Größe der Öffnungen variiert, so variiert auch die Größe von Partikeln, welche im Zuge der Aerosolbildung gebildet werden. Generell kann es bevorzugt sein, wenn die Austragsöffnungen einen Durchmesser zwischen ungefähr 0.25 und 6 µm besitzen. Auf diese Weise lassen sich Partikel mit einem Durchmesser von ungefähr 0.5 bis 12 µm erzeugen, welche besonders bevorzugt im Hinblick auf inhalative Anwendungen sind. Haben die Austragsöffnungen einen Durchmesser im Bereich von 0.25 bis 1 µm, so kann dadurch ein Aerosol erzeugt werden, welches Partikel mit einem Durchmesser im Bereich von 0.5 µm bis 2 µm aufweist. Dieser Partikeldurchmesser ist besonders vorteilhaft für eine Zuführung der erfindungsgemäßen Zusammensetzung, insbesondere des Antidepressivums, zum Alveolengang und zu den Lungenbläschen (Alveolen). Austragsöffnungen mit einem Durchmesser von ungefähr 1 bis 2 µm können Partikel mit einem Durchmesser von ungefähr 2 bis 4 µm erzeugen. Diese Partikeldurchmesser sind für eine Zuführung der erfindungsgemäßen Zusammensetzung, insbesondere des Antidepressivums, oberhalb des Alveolenganges aber unterhalb der kleinen Bronchien von besonderem Vorteil. Austragsöffnungen mit einem Durchmesser von 2 bis 4 µm können dagegen Partikel mit einem Durchmesser von 4 bis 8 µm erzeugen. Diese Partikeldurchmesser erlauben dagegen mit besonderem Vorteil eine Zuführung der erfindungsgemäßen Zusammensetzung, insbesondere des Antidepressivums, in den Atmungstrakt aufsteigend von den drückliche Bezugnahme zum Inhalt der vorliegenden Beschreibung gemacht wird.

Weiterhin wird eine Patrone bzw. Kartusche, insbesondere zur Verwendung in der intrapulmonalen Wirkstofffreisetzung, offenbart, umfassend:
- ein Behältnis mit einer Düsenöffnung und
- eine aerosolierbare bzw. versprühbare, inhalierbare pharmazeutische Zusammensetzung, umfassend zumindest ein Dispersionsmittel und/oder zumindest ein pharmazeutisch verträgliches Trägermaterial und zumindest ein Antidepressivum.

Behälter bzw. Behältnisse, welche verwendet werden können, können grundsätzlich unterschiedliche Formen aufweisen. Beispielsweise kann es sich bei den Behältern um einzeldosierte Verpackungen, beispielsweise Blisterverpackungen, welche vorzugsweise eine flüssige und sterile Zusammensetzung enthalten, handeln. Die Behälter können weiterhin zumindest ein Volumen von ungefähr 0.035 bis 0.095 cm3, insbesondere 0.045 bis 0.070 cm3, aufweisen. Es kann zudem möglich sein, dass das Behältnis eine Kapsel ist, welche ein trockenes Pulver, enthaltend ein Antidepressivum, enthält.

Außerdem wird auch ein Verfahren zur Prophylaxe und/oder symptomatischen Behandlung von zystischer Fibrose, insbesondere zur Prophylaxe und/oder Behandlung von bei zystischer Fibrose auftretenden Infektionen und/oder Infektionserkrankungen, offenbart, wobei die erfindungsgemäße Zusammensetzung, insbesondere in einer therapeutisch wirksamen Menge, einem Patienten verabreicht wird. Bei dem Patienten kann es sich um einen Menschen oder um ein Tier, beispielsweise Pferd, Rind, Schwein, Affe, Ratte, Maus oder Hamster, handeln. Bevorzugt kommt das Verfahren jedoch beim Menschen zur Anwendung. Das Verfahren ist grundsätzlich gleichermaßen für männliche wie weibliche Patienten geeignet. Auch Kinder können mit Hilfe des Verfahrens behandelt werden.

In einer bevorzugten Ausführungsform wird die Zusammensetzung intrapulmonal, vorzugsweise inhalativ, verabreicht. Zur intrapulmonalen, vorzugsweise inhalativen, Verabreichung kommt in der Regel eine hierfür geeignete Verabreichungs- bzw. Applikationshilfe, insbesondere ein Inhalator, zum Einsatz. Als Verabreichungshilfe kann beispielsweise eine Sprüh- oder Sprayvorrichtung verwendet werden. Bevorzugt wird die erfindungsgemäße Zusammensetzung für die inhalative Verabreichung zerstäubt bzw. aerolisiert.

Offenbart wird weiterhin auch ein Verfahren zur Inhibierung der sauren Sphingomyelinase in den Lungen eines Patienten, umfassend die folgenden Schritte:
- Aerosolieren bzw. Zerstäuben (Versprühen) einer pharmazeutischen Zusammensetzung, umfassend zumindest ein Dispersionsmittel und/oder zumindest ein pharmazeutisch verträgliches Trägermaterial und zumindest ein Antidepressivum, und
- Inhalieren der aeroslierten bzw. zerstäubten (versprühten) Zusammensetzung in die Lungen des Patienten.

Außerdem wird auch ein Verfahren zur Verringerung der Ceramid-Konzentration in den Lungen eines Patienten offenbart, umfassend die folgenden Schritte:
- Aerosolieren bzw. Zerstäuben (Versprühen) einer pharmazeutischen Zusammensetzung, umfassend zumindest ein Dispersionsmittel und/oder zumindest ein pharmazeutisch verträgliches Trägermaterial und zumindest ein Antidepressivum, und
- Inhalieren der aeroslierten bzw. zerstäubten (versprühten) Zusammensetzung in die Lungen des Patienten.

Schließlich wird ein Verfahren zur symptomatischen Behandlung von zystischer Fibrose, insbesondere zur Behandlung von bei zystischer Fibrose auftretenden Infektionen und/oder Infektionserkrankungen, offenbart, umfassend die folgenden Schritte:
- Diagnostizieren von zystischer Fibrose bei einem Patienten,
- Aerosolieren bzw. Zerstäuben (Versprühen) einer pharmazeutischen Zusammensetzung, umfassend zumindest ein Antidepressivum und vorzugsweise zumindest ein Dispersionsmittel und/oder zumindest ein pharmazeutisch verträgliches Trägermaterial,
- Inhalieren der aerosolierten bzw. zerstäubten (versprühten) Zusammensetzung in die Lungen des Patienten und
- Ablagern lassen einer ausreichenden Menge von Partikeln, umfassend zumindest ein Antidepressivum, auf den Lungen des Patienten, um die zystische Fibrose zu behandeln.

Die Zusammensetzung wird mit besonderem Vorteil derart aerosoliert bzw. versprüht, dass Partikel entstehen, welche sich ohne Weiteres in ein Lungengewebe inhalieren lassen, ohne dass die Partikel wieder exhaliert werden. Bevorzugt weist die aerosolierte pharmazeutische Zusammensetzung bei den Verfahren Partikel mit einem Durchmesser, insbesondere aerodynamischem Durchmesser, zwischen 0,1 und 12 µm, insbesondere 1 und 12 µm, bevorzugt 2 und 12 µm, auf.

Das Antidepressivum wird vorzugsweise in einer Dosis verabreicht, die 1/10 bis 1/1000 einer systemisch, insbesondere oral oder intravenös, verabreichten Dosis des Antidepressivums entspricht. Besonders bevorzugt wird das Antidepressivum in einer täglichen Dosis zwischen 0,01 und 20 mg, insbesondere 0,1 und 10 mg, verabreicht.

Bezüglich weiterer Merkmale und Einzelheiten zu den erfindungsgemäßen Verfahren wird vollständig auf die bisherige Beschreibung verwiesen.

Die genannten Merkmale und weitere Merkmale der Erfindung ergeben sich aus der nachfolgenden Beschreibung von Beispielen in Verbindung mit den Unteransprüchen und Figuren. Hierbei können die einzelnen Merkmale jeweils für sich oder zu mehreren in Kombination miteinander verwirklicht sein. Die Figuren werden hiermit durch ausdrückliche Bezugnahme zum Inhalt der Beschreibung gemacht.

In den Figuren zeigen schematisch:
- Figur 1:: den Einfluss verschiedener Antidepressiva auf die Aktivität der saueren Sphingomyelinase bei Cftr-defizienten Mäusen im Vergleich zu unbehandelten Cftr-defizienten Mäusen und unbehandelten Wildtyp-Mäusen,
- Figur 2:: den Einfluss verschiedener Antidepressiva auf die zelluläre Ceramid-Konzentration bei Cftr-defizienten Mäusen im Vergleich zu unbehandelten Cftr-defizienten Mäusen und unbehandelten Wildtyp-Mäusen,
- Figur 3:: den Einfluss verschiedener Antidepressiva auf die Infektionsanfälligkeit bei Cftr-defizienten Mäusen im Vergleich zu unbehandelten Cftr-defizienten Mäusen und unbehandelten Wildtyp-Mäusen,
- Figur 4:: den Einfluss verschiedener Antidepressiva auf die pulmonale Konzentration des Entzündungsmediators Interleukin-1β bei Cftr-defizienten Mäusen im Vergleich zu unbehandelten Cftr-defizienten Mäusen und unbehandelten Wildtyp-Mäusen,
- Figur 5:: den Einfluss von Amitriptylin in verschiedenen Verabreichungsformen auf die Aktivität der sauren Sphingomyelinase bei Cftr-defizienten Mäusen und Wildtyp-Mäusen,
- Figur 6:: den Einfluss von Amitriptylin in verschiedenen Verabreichungsformen auf die zelluläre Ceramid-Konzentration bei Cftr-defizienten Mäusen und Wildtyp-Mäusen,
- Figur 7:: den Einfluss von Amitriptylin in verschiedenen Verabreichungsformen auf die Infektionsanfälligkeit bei Cftr-defizienten Mäusen und Wildtyp-Mäusen.

### 1. Methoden

### 1.1 Cftr-defiziente Mäuse

Die Cftr-defizienten Mäuse wurden ursprünglich von Jackson Laboratories (Bar Harbour, Maine, USA) bezogen und dann bei der Firma Cycnad selbst gezüchtet. Die verwendeten Mäuse waren defizient bezüglich des Cftr. Hierbei handelt es sich um das Mäuse-Äquivalent zu dem humanen CFTR. Die verwendeten Versuchstiere exprimierten jedoch den humanen CFTR im Darm. Dies wurde unter der Kontrolle eines Promotors für Fettsäure bindendes Protein (FABP, Fatty Acid Binding Protein) erreicht. Dieser Promotor vermittelt eine spezifisch intestinale Expression von CFTR. Die verwendeten Mäuse besaßen einen gemischten genetischen Hintergrund, bestehend aus C57BL/6, FVB/N und 129.

Zur Herstellung weiterer Cftr-Knockout-Mäuse wurden bezüglich B6.129P2(CF/3)-Cftr^{TgH(neoim)Hgu} (abgekürzt: Cftr^{MHH}) gen-gleichartige Mäuse verwendet. Der durch Inzucht erzeugte CF-Stamm CF/3-Cftr^{TgH(neoim)Hgu} wurde durch eine strikte Paarung von Bruder- und Schwestertieren gezüchtet, ausgehend von einer mutierten Maus des ursprünglichen Typs Cftr^{TgH(neoim)Hgu}. Letztere wurde durch eine sogenannte Insertions-Mutagenese in dem Cftr-Exon10 hergestellt. Die Maus produzierte geringe Mengen Cftr.

Des Weiteren wurde ein Gen-gleichartiger Stamm des Typs Cftr^{MHH} erzeugt, indem eine Rückkreuzung der Zielmutation in den durch Inzucht erzeugten Hintergrund B6 vorgenommen wurde. Dabei wurden Genidentische B6-Mäuse als Kontrolltiere verwendet. Der hygienische Zustand der Versuchstiere wurde regelmäßig entsprechend den 2002 erlassenen Empfehlungen der FELASA (Federation of European Laboratory Animal Science Associations) überprüft.

### 1.2 Inhalation

Die Inhalation wurde mit Hilfe eines Inhalators (Pariboy® SX) durchgeführt. Das flüssige Reservoir war bei diesem Inhalator mit einer Luftdüse verbunden, wodurch ein feines Aerosol erzeugt werden konnte, welches von den Mäusen über eine Maske inhaliert wurde. Hierzu wurden die Nasen der Versuchstiere für 10 Minuten manuell in die Öffnung der Maske gedrückt. Dies erlaubte eine hervorragende Kontrolle des Inhalationsprozesses. Alle Mäuse wurden im Abstand von 12 Stunden einem 3-maligen Inhalationsprozess unterworfen.

### 1.3 Die Bestimmung der Aktivität der sauren Sphingomyelinase

Die Lungen wurden Schock gefroren, unter flüssigem Stickstoff homogenisiert und anschließend in einem Puffer aus 250 mM Natriumacetat (pH 5.0), 1.3 mM EDTA und 1 % NP40 überführt. Danach wurde das Homogenisat insgesamt drei Mal einer Ultraschallbehandlung unterworfen (ultrasonic processor tip sonicator). Anschließend wurde ein Aliquot der Proben mit 250 mM Natriumacetat (pH 5.0), 1.3 mM EDTA 0.1 % NP40 verdünnt und mit 0.05 µCi [¹⁴C]Sphingomyelin pro Probe inkubiert (52 mCi/mmol; MP Bio-medicals Irvine CA, USA). Die radioaktive Probe von [¹⁴C]Sphingomyelin wurde getrocknet, in 250 mM Natriumacetat (pH 5.0), 1.3 mM EDTA und 0.1 % NP40 resuspendiert und anschließend für 10 Minuten einer Ultraschallbehandlung unterworfen, bevor sie zu den homogenisierten Proben hinzugegeben wurde. Die Proben wurden insgesamt während 30 Minuten bei ca. 37°C inkubiert. Anschließend wurde die enzymatische Reaktion durch Extraktion beendet (Extraktion mit einem Lösungsmittelgemisch aus Chloroform und Methanol im Volumenverhältnis Chloroform/Methanol 2:1). Danach wurden die Proben zentrifugiert und jeweils ein Aliquot der wässrigen Phase einer Szintillationszählung unterworfen, um die Freisetzung von [¹⁴C]Phosphorylcholin aus [¹⁴C]Sphingomyelin zu bestimmen.

### 1.4 Messung der Ceramid-Konzentration

Nach Tötung der Versuchstiere wurden die Lungen entnommen, unter flüssigem Stickstoff homogenisiert und in eine Mischung aus Chloroform, Methanol und 1 N Salzsäure (100:100:1, v/v/v) zugegeben. Nach Zugabe von 200 µl H₂O wurden die Proben während 5 Minuten zentrifugiert (14.000 rpm, rotations per minute). Danach wurde die untere Phase gesammelt, getrocknet und einer alkalischen Hydrolyse von Diacylglycerol in 0.1 N methanolischer Kalilauge bei 37 °C für 60 Minuten unterworfen. Die Proben wurden erneut extrahiert, die untere Phase wurde getrocknet und anschließend in 20 µl einer Detergenz-Lösung resuspendiert (7.5 % (w/v) n-Octylglucopyranosid, 5 mM Cardiolipin in 1 mM Diethylentriaminpentaessigsäure (DTPA)). Nach einer Ultraschallbehandlung (10 Minuten) wurden die Proben zu 70 µl einer Reaktionsmischung, welche 10 µl Diacylglycerol-Kinase (GE Healthcare Europe, München, Germany), 0.1 M Imidazol/ HCl (pH 6.6), 0.2 mM Diethylentriaminpentaessigsäure (pH 6.6), 70 mM Natriumchlorid, 17 mM Magnesiumchlorid, 1.4 mM EGTA, 2 mM DTT, 1 µM ATP und 10 µCi [³²P]γATP enthielt, hinzugegeben. Die Kinasereaktion wurde während 30 Minuten bei Raumtemperatur durchgeführt und anschließend durch Zugabe von 1 ml Chloroform:Methanol:1 N HCl (100:100:1, v/v/v), 170 µl gepufferte Salzlösung (135 mM Natriumchlorid, 1.5 mM Calciumchlorid, 0.5 mM Magnesiumchlorid, 5.6 mM Glucose, 10 mM HEPES, pH 7.2) und 30 µl einer 100 mM EDTA-Lösung beendet. Die Proben wurden danach gevortext und die entstandenen Phasen getrennt. Die untere Phase wurde gesammelt, getrocknet, in 20 µl eines Chloroform/Methanol-Gemisches (1:1, v/v) gelöst und durch Dünnschichtchromatographie aufgetrennt (Silika G 60 TLC Platten, Chloroform:Methanol:Essigsäure (65:15:5, v/v/v)). Danach wurden die Dünnschichtchromatographie-Platten jeweils mit einem Röntgenfilm belegt. Die Ceramid-Spots wurden anhand einer Co-Migration eines C₁₆-Ceramidstandards identifiziert. Der Einbau von [³²P] in Ceramid wurde mittels einer Flüssigszintillationszählung quantifiziert, nachdem die Spots von den Platten verschwunden waren. Die Ceramid-Mengen wurden durch Vergleich mit einer Standardkurve unter Verwendung von C₁₆-Ceramid als Substrat bestimmt.

### 1.5 Infektion

Die Bakterien wurden für 14 bis 14.5 Stunden auf TSA Agarplatten gezüchtet (Becton Dickinson). Frische Platten wurden hierfür maximal für 2 Wochen verwendet. Danach wurden die Bakterien in Erlenmeyerkolben in 40 ml eines vorgewärmten und sterilen Puffers (TSB, Becton Dickinson) überführt. Die optische Dichte (OD) bzw. Extinktion wurde auf 0.225 (entspricht 5.5x10⁸ CFU/ml) eingestellt und die Bakterien wurden für 60 Minuten bei 37°C unter Schütteln bei 125 rpm inkubiert. Die Qualität der Agarplatten und von TSB sowie die Schüttelgeschwindigkeit waren entscheidend für den Erhalt von reproduzierbaren Ergebnissen. Auf diese Weise konnte eine Pseudomonas aeruginosa Population exakt in der frühen Log-Phase erhalten werden (die Bakterien vermehrten sich ungefähr um das 10-fache innerhalb einer Inkubationsdauer von 60 Minuten). Die Tatsache, dass es sich bei den Bakterien um Bakterien der frühen Log-Phase handelte, war äußerst wichtig, um für die anschlie-ßende Infektion mit den Versuchstieren reproduzierbare Bedingungen zu erhalten. Die Bakterien wurden bei 2800 rpm (1600xg) in einem 50 ml Röhrchen zentrifugiert. Danach wurde der Überstand sorgfältig entfernt und die Bakterien in 10 ml RPMI-1640, ergänzt mit 10 mM HEPES (pH 7.3), resuspendiert. Die Suspension wurde in ein 15 ml Röhrchen überführt und anschließend zweimal gewaschen. Schließlich wurden die Bakterien in vorgewärmtem HEPES/Salz (HS; 132 mM NaCl, 20 mM HEPES (pH 7.4), 5 mM KCI, 1 mM CaCl₂, 0.7 mM MgCl₂, 0.8 mM MgSO₄) resuspendiert und die bakterielle optische Dichte bestimmt. Danach wurden die Bakterien mit vorgewärmtem H/S auf eine Konzentration von 10⁸ CFU/20 µl verdünnt. Die Mäuse wurden dann innerhalb der nächsten 10 Minuten infiziert. Zu diesem Zweck wurde eine Spritze mit einem Volumen von 1 ml und einer 30-Gauge Nadel verwendet. Die Mäuse wurden für 10 bis 15 Sekunden in Ether anästhesiert. Im Anschluss daran wurde die Nadel sofort 2 mm weit in die Nasen der Mäuse eingeführt und die Bakterien vorsichtig injiziert. Zwischen der ersten und der letzten Maus, welche infiziert wurden, ließ man maximal ein Zeitintervall von 10 Minuten verstreichen, um auf diese Weise Schwankungen bei der Entstehung von lebensfähigen Bakterienkolonien zu vermeiden. Gewöhnlich wurden nicht mehr als 10 Mäuse pro Experiment infiziert. Außerdem wurde nie mehr als 1 Maus pro Versuchsgruppe infiziert. Die Mäuse wurden 2 Stunden nach der Applikation von Pseudomonas aeruginosa getötet. Anschließend wurde die Zahl der Bakterien in der Lunge als Maß für die Empfindlichkeit der Mäuse, an einer Pseudomonas aeruginosa-Infektion zu erkranken, gemessen. Dazu wurde die Lunge entnommen, mechanisch homogenisiert, intrazelluläre Bakterien durch eine 10-minütige Behandlung mit 5 mg/ml Saponin bei 37 °C freigesetzt, die Proben mit 10 ml PBS (phosphate buffered saline) gewaschen, die Pellets in PBS aufgenommen und Aliqots davon auf TSA-Agarplatten ausplattiert. Die Bakterienkolonien wurden nach 15 Stunden Wachstum bei 37 °C als Maß für die Anzahl pulmonaler Bakterien und damit als Maß für eine Infektion gezählt.

### 1.6 Messung der Ausschüttung von Interleukin-1β

Um die Konzentration von Interleukin-1β zu bestimmen, wurden die Lungen 60 Minuten nach der letzten Inhalation entfernt, unter flüssigem Stickstoff homogenisiert und für 10 Minuten in 125 mM NaCl, 25 mM TrisHCl (pH 7.4), 10 mM EDTA, 10 mM Natriumpyrophospat, 3% NP40, 10 Mikrogramm/ml Aprotinin und 10 Mikrogramm/ml Leupeptin lysiert. Danach wurde ein Aliquot auf Interleukin-1β analysiert. Hierfür kam ein kommerziell erhältlicher ELISA für Interleukin-1β zum Einsatz. Dabei wurde der Assay exakt nach dem Protokoll des Anbieters durchgeführt.

### 2. Versuche

### 2.1 Bestimmung des Einflusses verschiedener Antidepressiva auf die Aktivität von saurer Sphingomyelinase in Lungenzellen

Wildtyp-Mäuse und Cftr-defiziente Mäuse (Cftr-Knock-out-Mäuse) inhalierten eine Lösung von 4 mg/l Amitriptylin, 12.5 mg/l Trimipramin, 9 mg/l Desipramin, 8 mg/l Chlorprothixen, 4.5 mg/l Fluoxetin, 5mg/l Amlodipin und 35 mg/l Sertralin. Alle Substanzen wurden hierfür in Wasser oder DMSO gelöst und anschließend mindestens 1:100 (für DMSO: mindestens 1:1000) in 0.9% Natriumchloridlösung verdünnt. Die Mäuse inhalierten die Lösungen für 10 Minuten. Für die Inhalation wurde ein Inhalator (Pariboy^{®}) verwendet. Innerhalb der Inhalationszeit wurden ungefähr 2 ml der verabreichten Lösungen zerstäubt. Die Inhalation wurde insgesamt dreimal in Intervallen von 12 Stunden durchgeführt. 60 Minuten nach der letzten Inhalation wurden die Lungen entnommen, und die Aktivität der sauren Sphingomyelinase wurde in den Lungenextrakten bestimmt.

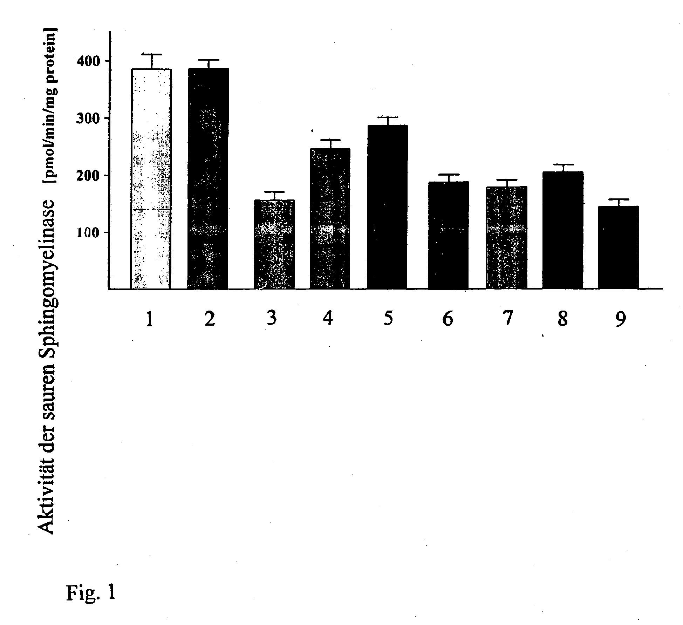

Die Ergebnisse sind grafisch in der Fig. 1 dargestellt. Auf der Ordinate ist dabei die Aktivität der sauren Sphingomyelinase [pmol/min/mg Protein] wiedergegeben. Auf der Abszisse sind die mit den vorstehend genannten Antidepressiva-Lösungen behandelten Cftr-defizienten Mäuse im Vergleich zu unbehandelten Cftr-defizienten Mäusen sowie unbehandelten Wildtyp-Mäusen wiedergegeben. Auf der Abszisse bedeuten die Ziffern:
- 1:: Cftr-defiziente Mäuse, unbehandelt;
- 2:: Wildtyp-Mäuse, unbehandelt;
- 3:: Cftr-defiziente Mäuse nach Inhalation einer Amitriptylinlösung (4 mg/l);
- 4:: Cftr-defiziente Mäuse nach Inhalation einer Trimipramin-Lösung (12.5 mg/l);
- 5:: Cftr-defiziente Mäuse nach Inhalation einer Desipramin-Lösung (9 mg/l);
- 6:: Cftr-defiziente Mäuse nach Inhalation einer Amlodipin-Lösung (5 mg/l);
- 7:: Cftr-defiziente Mäuse nach Inhalation einer Sertralin-Lösung (35 mg/l);
- 8:: Cftr-defiziente Mäuse nach Inhalation einer Fluoxetin-Lösung (4.5 mg/l);
- 9:: Cftr-defiziente Mäuse nach Inhalation einer Chlorprothixen-Lösung (8 mg/l).

Die in Fig. 1 grafisch dargestellten Ergebnisse zeigen, dass bei den behandelten Cftr-defizienten Mäusen eine signifikante Verringerung der Aktivität der sauren Sphingomyelinase erreicht werden konnte.

### 2.2 Bestimmung des Einflusses verschiedener Antidepressiva auf die pulmonale Ceramid-Konzentration

Wildtyp-Mäuse und Cftr-defiziente Mäuse wurden mit einer Lösung von 4 mg/l Amitryptilin, 12.5 mg/l Trimipramin, 9 mg/l Desipramin, 8 mg/l Chlorprothixen, 4.5 mg/l Fluoxetin, 5 mg/l Amlodipin und 35 mg/l Sertralin inhaliert. Die Substanzen wurden hierfür in Wasser oder DMSO gelöst und anschließend wie oben in 0.9% Natriumchlorid-Lösung verdünnt. Die Mäuse inhalierten die vorstehend aufgeführten Antidepressiva-Lösungen über einen hierfür geeigneten Inhalator (Pariboy^{®}). Während der Inhalationszeit wurden ungefähr 2 ml der verabreichten Lösungen zerstäubt. Die Inhalation wurde insgesamt dreimal in Abständen von 12 Stunden durchgeführt. 60 Minuten nach der Inhalation wurden die Lungen aus den Versuchstieren entnommen und die pulmonale Ceramid-Konzentration in den Lungenextrakten bestimmt.

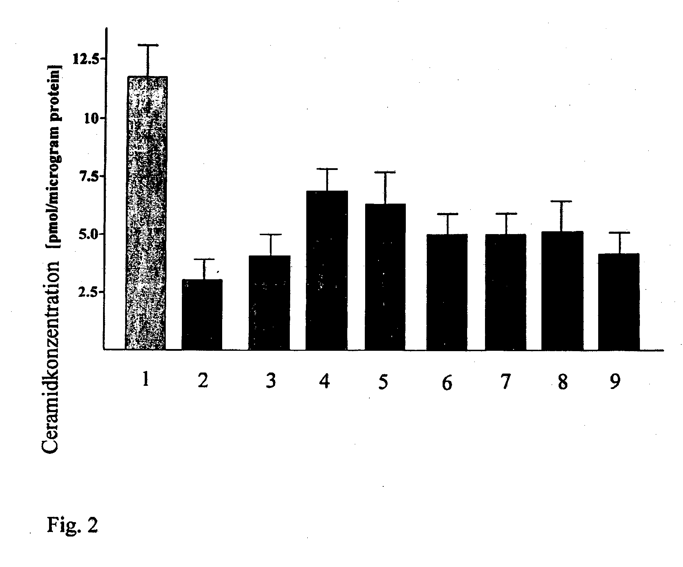

Die Ergebnisse sind grafisch in Fig. 2 dargestellt. Dort ist auf der Ordinate die pulmonale Ceramid-Konzentration [pmol/microgramm Protein] aufgetragen. Auf der Abszisse sind die mit den einzelnen Antidepressiva-Lösungen behandelten Cftr-defizienten Mäuse im Vergleich zu unbehandelten Cftr-defizienten Mäusen und unbehandelten Wildtyp-Mäusen dargestellt. Auf der Abszisse bedeuten die Ziffern:
- 1:: Cftr-defiziente Mäuse, unbehandelt;
- 2:: Wildtyp-Mäuse, unbehandelt;
- 3:: Cftr-defiziente Mäuse nach Inhalation einer Amitriptylinlösung (4 mg/l);
- 4:: Cftr-defiziente Mäuse nach Inhalation einer Trimipramin-Lösung (12.5 mg/l);
- 5:: Cftr-defiziente Mäuse nach Inhalation einer Desipramin-Lösung (9 mg/l);
- 6:: Cftr-defiziente Mäuse nach Inhalation einer Amlodipin-Lösung (5 mg/l);
- 7:: Cftr-defiziente Mäuse nach Inhalation einer Sertralin-Lösung (35 mg/l);
- 8:: Cftr-defiziente Mäuse nach Inhalation einer Fluoxetin-Lösung (4.5 mg/l);
- 9:: Cftr-defiziente Mäuse nach Inhalation einer Chlorprothixen-Lösung (8 mg/l).

Die in Figur 2 grafisch dargestellten Ergebnisse zeigen, dass durch eine inhalative Verabreichung der oben aufgeführten Antidepressiva-Lösungen bei Cftr-defizienten Mäusen (Mäuse, die an zystischer Fibrose erkrankt sind) eine weitgehende Normalisierung der pulmonalen Ceramid-Konzentration erzielt werden konnte.

### 2.3 Bestimmung des Einflusses verschiedener Antidepressiva auf die Infektionsanfälligkeit gegenüber Pseudomonas aeruginosa

Wildtyp-Mäuse und Cftr-defiziente Mäuse wurden mit einer Lösung von 4 mg/l Amitryptilin, 12.5 mg/l Trimipramin, 9 mg/l Desipramin, 8 mg/l Chlorprothixen, 4.5 mg/l Fluoxetin, 5 mg/l Amlodipin und 35 mg/l Sertralin inhaliert. Die Substanzen wurden hierfür in Wasser oder DMSO gelöst und wie oben in 0.9% Natriumchlorid-Lösung verdünnt. Die Mäuse inhalierten die vorstehend aufgeführten Antidepressiva-Lösungen über einen hierfür geeigneten Inhalator (Pariboy®). Während der Inhalationszeit wurden ungefähr 2 ml der verabreichten Lösungen zerstäubt. Die Inhalation wurde insgesamt dreimal in Abständen von 12 Stunden durchgeführt. 60 Minuten nach der Inhalation wurden die Lungen aus den Versuchstieren entfernt und die Infektionsanfälligkeit der Versuchstiere gegenüber Pseudomonas aeruginosa bestimmt.

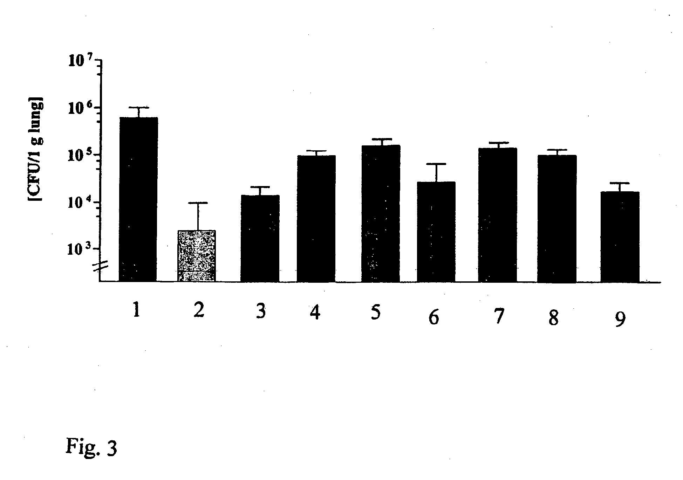

Die hierbei gewonnenen Ergebnisse sind schematisch in Fig. 3 wiedergegeben. Dort ist auf der Ordinate die Anzahl der koloniebildenden Einheiten (CFU) von Pseudomonas aeruginosa pro g Lungengewebe wiedergegeben. Auf der Abszisse sind die mit den einzelnen Antidepressiva-Lösungen behandelten Cftr-defizienten Mäuse im Vergleich zu unbehandelten Cftr-defizienten Mäusen und unbehandelten Wildtyp-Mäusen dargestellt. Auf der Abszisse bedeuten die Ziffern:
- 1:: Cftr-defiziente Mäuse, unbehandelt;
- 2:: Wildtyp-Mäuse, unbehandelt;
- 3:: Cftr-defiziente Mäuse nach Inhalation einer Amitriptylinlösung (4 mg/l);
- 4:: Cftr-defiziente Mäuse nach Inhalation einer Trimipramin-Lösung (12.5 mg/l);
- 5:: Cftr-defiziente Mäuse nach Inhalation einer Desipramin-Lösung (9 mg/l);
- 6:: Cftr-defiziente Mäuse nach Inhalation einer Amlodipin-Lösung (5 mg/l);
- 7:: Cftr-defiziente Mäuse nach Inhalation einer Sertralin-Lösung (35 mg/l);
- 8:: Cftr-defiziente Mäuse nach Inhalation einer Fluoxetin-Lösung (4.5 mg/l);
- 9:: Cftr-defiziente Mäuse nach Inhalation einer Chlorprothixene-Lösung (8 mg/l).

Die in Fig. 3 grafisch dargestellten Ergebnisse zeigen, dass bei inhalativer Behandlung von Cftr-Knockout-Mäusen mit den oben aufgeführten Antidepressiva-Lösungen eine weitgehende Normalisierung der Infektionsanfälligkeit der Versuchstiere gegenüber einer Infektion mit Pseudomonas aeruginosa erreicht werden konnte. Hierbei inhalierten die Versuchstiere die Antidepressiva-Lösungen 60 Minuten vor einer Infektion mit Pseudomonas aeruginosa. Die Fig. 3 grafisch dargestellten Ergebnisse zeigen, dass die Versuchstiere nahezu resistent gegen eine Infektion durch Pseudomonas aeruginosa sind, im Gegensatz zu unbehandelten Cftr-defizienten Mäusen. Letztere stellten sich als in hohem Maße anfällig für eine Infektion durch Pseudomonas aeruginosa heraus. Dies belegt die prophylaktische Wirkung der erfindungsgemäßen Zusammensetzungen im Hinblick auf eine mögliche Infektion durch Pseudomonas aeruginosa.

### 2.4 Bestimmung des Einflusses von verschiedenen Antidepressiva auf die Ausschüttung von Interleukin-1β

Wildtyp-Mäuse und Cftr-defiziente Mäuse wurden mit einer Lösung von 4 mg/l Amitryptilin, 12.5 mg/l Trimipramin, 9 mg/l Desipramin, 8 mg/l Chlorprothixen, 4.5 mg/l Fluoxetin, 5 mg/l Amlodipin und 35 mg/l Sertralin inhaliert. Die Substanzen wurden hierfür in Wasser oder DMSO gelöst und anschließend wie oben in 0.9% Natriumchlorid-Lösung verdünnt. Die Mäuse inhalierten die vorstehend aufgeführten Antidepressiva-Lösungen über einen hierfür geeigneten Inhalator (Pariboy®). Während der Inhalationszeit wurden ungefähr 2 ml der verabreichten Lösungen zerstäubt. Die Inhalation wurde insgesamt dreimal in Abständen von 12 Stunden durchgeführt. 60 Minuten nach der Inhalation wurden die Lungen aus den Versuchstieren entfernt und die Konzentration von Interleukin-1β (IL-1β) im Lungengewebe bestimmt.

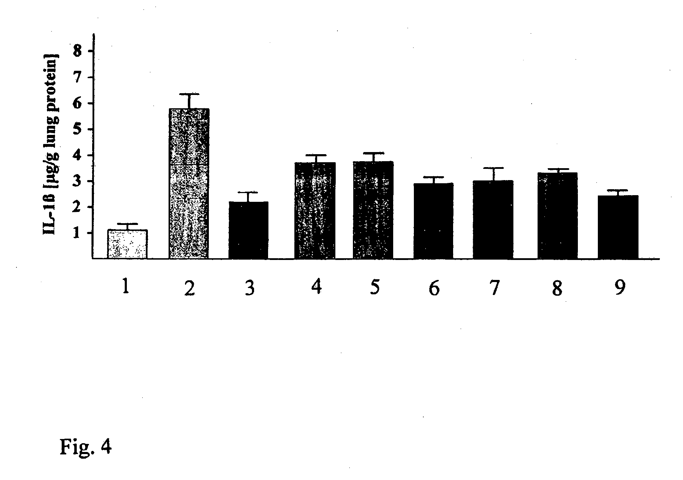

Die Ergebnisse sind grafisch in Fig. 4 wiedergegeben. Dort ist auf der Ordinate die Konzentration von Interleukin-1β (µg/g Lungenprotein) angegeben. Auf der Abszisse sind mit den Antidepressiva-Lösungen behandelte Cftr-defizienten Mäuse im Vergleich zu unbehandelten Cftr-defizienten Mäusen und unbehandelten Wildtyp-Mäusen dargestellt. Auf der Abszisse bedeuten die Ziffern:
- 1:: Cftr-defiziente Mäuse unbehandelt;
- 2:: Wildtyp-Mäuse unbehandelt;
- 3:: Cftr-defiziente Mäuse nach Inhalation einer Amitriptylinlösung (4 mg/l);
- 4:: Cftr-defiziente Mäuse nach Inhalation einer Trimipramin-Lösung (12.5 mg/l);
- 5:: Cftr-defiziente Mäuse nach Inhalation einer Desipramin-Lösung (9 mg/l);
- 6:: Cftr-defiziente Mäuse nach Inhalation einer Amlodipin-Lösung (5 mg/l);
- 7:: Cftr-defiziente Mäuse nach Inhalation einer Sertralin-Lösung (35 mg/l);
- 8:: Cftr-defiziente Mäuse nach Inhalation einer Fluoxetin-Lösung (4.5 mg/l);
- 9:: Cftr-defiziente Mäuse nach Inhalation einer Chlorprothixen-Lösung (8 mg/l).

### 2.5 Einfluss verschiedener Verabreichungsformen am Beispiel einer Amitritryptilin-Lösung

Wildtyp-Mäusen und Cftr-defizienten Mäusen wurden jeweils 1 ml einer Amitriptylin-Lösung (4 mg/l H₂O) während 20 Minuten mit Hilfe einer apothekenüblichen Inhalationsvorrichtung inhaliert. Die tatsächlich verabreichte Menge an Amitryptilin betrug daher ca. 4 µg. Parallel dazu wurden anderen Wildtyp-Mäusen und Cftr-defizienten Mäusen jeweils 100 µl einer Amitriptylin-Lösung (2,5 g/l H₂O) per Injektion verabreicht, so dass die tatsächlich verabreichte Menge an Amitriptylin bei diesen Tieren 250 µg betrug. Die Injektionen wurden zwei Mal täglich über 2 Tage verabreicht.

Im Falle der inhalativen Behandlung wurden die Lungen der Mäuse 60 Minuten oder 12 Stunden nach Beendigung der Inhalation extrahiert, wobei im Falle der 12-stündigen Versuche die Inhalation nach 6 Stunden wiederholt wurde. Im Falle der durch eine Injektionslösung behandelten Mäuse wurden deren Lungen 12 Stunden nach der letzten Injektion entnommen.

Die Lungenextrakte wurden jeweils auf die Aktivität der sauren Sphingomyelinase, die Ceramid-Konzentration sowie auf die Besiedelung durch Pseudomonas Aeruginosa hin untersucht.

Folgende Ergebnisse wurden dabei konkret erhalten:

### 2.5.1 Saure Sphingomyelinase-Aktivität

In den Lungenextrakten von unbehandelten Wildtyp-Mäusen wurde eine Enzym-Aktivität von 405 ± 19 pMol/min/mg Protein festgestellt. Die Lungenextrakte von unbehandelten, Cftr-defizienten Mäuse wiesen eine Enzym-Aktivität von 375 ± 19 pMol/min/mg Protein auf.

In den nach 60 min extrahierten Lungen von durch Inhalation behandelten Wildtyp-Mäusen wurde eine Aktivität von 176 ± 18 pMol/min/mg Protein festgestellt. In den Lungenextrakten von entsprechend behandelten, Cftr-defizienten Mäusen wurde eine Enzym-Aktivität von 148 ± 17 pMol/min/mg Protein gemessen.

Die nach 12 Stunden extrahierten Lungen von inhalativ behandelten Wildtyp-Mäusen wiesen eine Enzym-Aktivität von 200 ± 14 pMol/min/mg Protein auf. Die Lungenextrakte von entsprechend behandelten, Cftr-defizienten Mäusen zeigten eine Enzym-Aktivität von 167 ± 9 pMol/min/mg Protein.

Die Lungen der durch eine Injektionslösung behandelten Wildtyp-Mäuse zeigten eine Enzym-Aktivität von 168 ± 19 pMol/min/mg Protein. Die Lungenextrakte von entsprechend behandelten, Cftr-defizienten Mäusen zeigten eine Enzym-Aktivität von 151 ± 17 pMol/min/mg Protein.

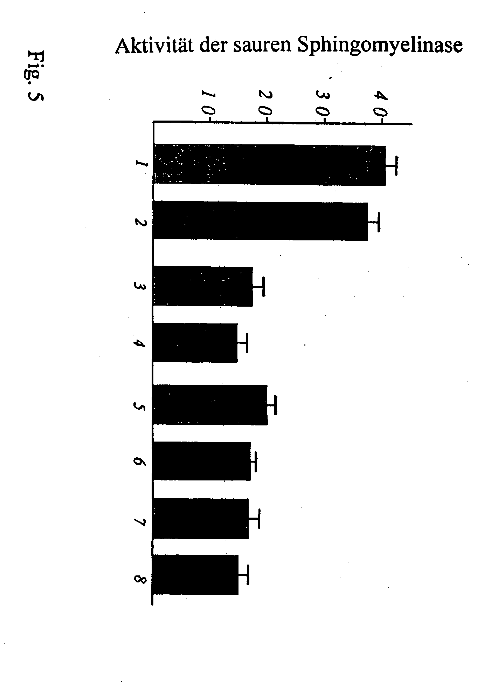

Diese Ergebnisse sind schematisch in Figur 5 wiedergegeben. Dort ist auf der Ordinate die Aktivität der sauren Sphingomyelinase, gemessen in pMol/min/mg Protein, aufgetragen. Auf der Abszisse sind die Wildtyp- und Cftr-defizienten Mäuse sowie die verschiedenen Verabreichungs- bzw. Darreichungsformen aufgeführt. Auf der Abszisse bedeuten die Ziffern:
- 1:: Wildtyp-Mäuse, unbehandelt;
- 2:: Cftr-defiziente Mäuse, unbehandelt;
- 3:: Wildtyp-Mäuse, Aktivität der sauren Sphingomyelinase 60 min nach Inhalation von 1 ml einer Amitriptylin-Lösung (4 mg/l);
- 4:: Cftr-defiziente Mäuse, Aktivität der sauren Sphingomyelinase 60 min nach Inhalation von 1 ml einer Amitriptylin-Lösung (4 mg/l);
- 5:: Wildtyp-Mäuse, Aktivität der sauren Sphingomyelinase 12 h nach Inhalation von 1 ml einer Amitriptylin-Lösung (4 mg/l);
- 6:: Cftr-defiziente Mäuse, Aktivität der sauren Sphingomyelinase 12 h nach Inhalation von 1 ml einer Amitriptylin-Lösung (4 mg/l);
- 7:: Wildtyp-Mäuse, Aktivität der sauren Sphingomyelinase 12 h nach intraperitonealer Injektion von 250 Mikrogramm Amitriptylin;
- 8:: Cftr-defiziente Mäuse, Aktivität der sauren Sphingomyelinase 12 h nach intraperitonealer Injektion von 250 Mikrogramm Amitriptylin.

Die Figur 5 macht deutlich, dass die erfindungsgemäße Zusammensetzung bei Vorliegen von zystischer Fibrose mit besonderem Vorteil eine signifikante Hemmung der sauren Sphingomyelinase bewirkt. Die Ergebnisse zeigen insbesondere, dass die Hemmung des Ceramid freisetzenden Enzyms zwischen 55 und 66%, bezogen auf die zelluläre Gesamtaktivität der sauren Sphingomyelinase in einer Cftr-defizienten Maus, betragen kann. Im Falle einer inhalativen Verabreichung der erfindungsgemäßen Zusammensetzung ist außerdem von Vorteil, dass diese Wirkung bereits mit niedrigen Amitriptylin-Dosen erreicht werden kann (effektiv verabreichte Dosis Amitriptylin bei der inhalativen Verabreichung betrug 4 µg). Dadurch können unerwünschte Nebenwirkungen, die mit zunehmenden Amitryptilin-Dosen gewöhnlich auftreten, vermieden werden.

### 2.5.2 Ceramid-Konzentration:

In den Lungenextrakten von unbehandelten Wildtyp-Mäusen konnte eine Ceramid-Konzentration von 2,3 ± 0,5 pMol/µg Protein gemessen werden. Die Lungenextrakte von unbehandelten, Cftr-defizienten Mäusen wiesen eine Ceramid-Konzentration von 16 ± 1,6 pMol/µg Protein auf.

In den nach 12 h extrahierten Lungen von Wildtyp-Mäusen wurde eine Ceramid-Konzentration von 1,6 ± 0,3 pMol/µg Protein gemessen. Die Lungenextrakte von entsprechend behandelten, Cftr-defizienten Mäusen wiesen eine Ceramid-Konzentration von 5,2 ± 0,79 pMol/µg Protein auf.

Die Lungenextrakte von parenteral behandelten Wildtyp-Mäusen wiesen eine Ceramid-Konzentration von 1,5 ± 0,4 pMol/µg Protein auf. Die Ceramid-Konzentration in den Lungenextrakten von entsprechend behandelten, Cftr-defizienten Mäusen betrug 4,9 ± 0,7 pMol/µg Protein.

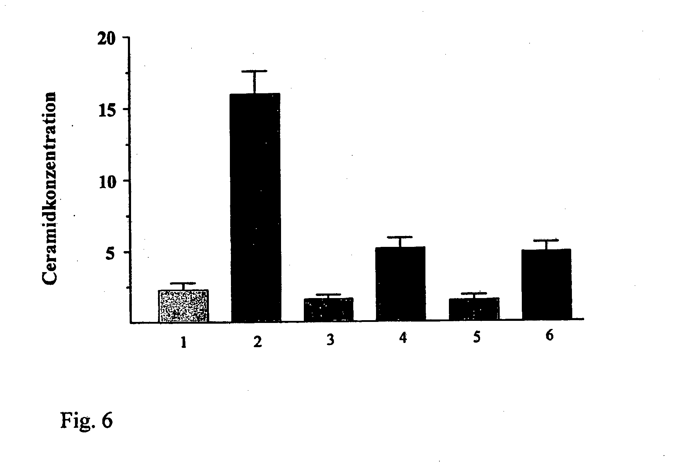

Die vorstehend aufgeführten Ergebnisse sind in der Figur 6 schematisch wiedergegeben. Dort ist auf der Ordinate die Ceramid-Konzentration, gemessen in pMol/µg Lungenprotein, angegeben. Auf der Abszisse sind die Wildtyp- und Cftr-defizienten Mäuse einschließlich der verschiedenen Verabreichungs- bzw. Darreichungsformen aufgeführt. Auf der Abszisse bedeuten die Ziffern:
- 1:: Wildtyp-Mäuse, unbehandelt;
- 2:: Cftr-defiziente Mäuse, unbehandelt;
- 3:: Wildtyp-Mäuse, Ceramidkonzentration 12 h nach Inhalation von 1 ml einer Amitriptylin-Lösung (4 mg/l);
- 4:: Cftr-defiziente Mäuse, Ceramidkonzentration 12 h nach Inhalation von 1 ml einer Amitriptylin-Lösung (4 mg/l);
- 5:: Wildtyp-Mäuse, Ceramidkonzentration 12 h nach intraperitonealer Injektion von 250 Mikrogramm Amitriptylin;
- 6:: Cftr-defiziente Mäuse, Ceramidkonzentration 12 h nach intraperitonealer Injektion von 250 Mikrogramm Amitriptylin.

Aus Figur 6 geht deutlich hervor, dass die erfindungsgemäße Zusammensetzung in besonderer Weise dafür geeignet ist, bei Vorliegen von zystischer Fibrose eine Normalisierung der Ceramid-Konzentration im Lungenepithel zu bewirken. Bezüglich weiterer Vorteile wird auf das bereits unter 2.5.1 Gesagte verwiesen.

### 2.5.3 Messung der Infektionsanfälligkeit für Pseudomonas aeruginosa

In diesen Versuchen wurden Wildtyp-Mäusen und Cftr-defizienten Mäusen 12 Stunden nach zweimaliger Inhalation mit jeweils 1 ml einer Amitriptylin-Lösung (4 mg/l H₂O) 1 x 10⁸ CFU (colony forming units) des Pseudomonas aeruginosa Stammes 762 in die Nase appliziert. Als Kontrollen dienten unbehandelte Mäuse. Die Mäuse wurden 2 Stunden nach der Applikation getötet. Anschließend wurde die Zahl der Bakterien in der Lunge als Maß für die Empfindlichkeit der Mäuse, an einer Pseudomonas aeruginosa-Infektion zu erkranken, gemessen. Dazu wurde die Lunge entnommen, mechanisch homogenisiert, intrazelluläre Bakterien durch eine 10-minütige Behandlung mit 5 mg/ml Saponin bei 37°C freigesetzt, die Proben mit 10 ml PBS (phosphate buffered saline) gewaschen, die Pellets in PBS aufgenommen und Aliquots davon auf TSA-Aggarplatten ausplattiert. Die Bakterienkolonien wurden nach 15 Stunden Wachstum bei 37°C als Maß für die Anzahl pulmonaler Bakterien und damit als Maß für eine Infektion gezählt. Dabei wurden die folgenden Ergebnisse erhalten:

In den Lungen von unbehandelten Wildtyp-Mäusen konnte eine Bakterienzahl von 4700 ± 2160 CFU gemessen werden. Die entnommenen Lungen von unbehandelten, Cftr-defizienten Mäusen wiesen eine Bakterienzahl von 6,3 x 10⁶ ± 1,87 x 10⁶ CFU auf. Die Lungen von durch Inhalation behandelten Wildtyp-Mäusen wiesen eine Bakterienzahl von 3600 ± 2892 CFU auf. In den Lungen von inhalativ behandelten, Cftr-defizienten Mäusen konnte eine Bakterienzahl von 12300 ± 4075 CFU festgestellt werden. Die Lungen von durch eine Injektionslösung behandelten Wildtyp-Mäusen wiesen eine Bakterienzahl von 2780 ± 1740 CFU auf. Die Lungen von entsprechend behandelten, Cftr-defizienten Mäusen wiesen eine Bakterienzahl von 11250 ± 2360 CFU auf.

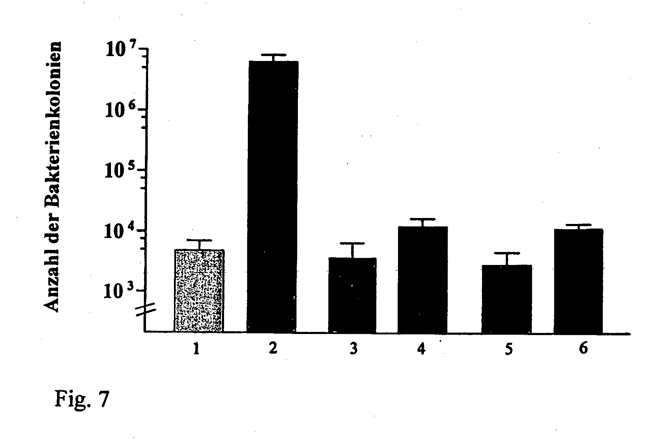

Die vorstehend genannten Ergebnisse sind graphisch in der Figur 7 wiedergegeben. Dort ist auf der Ordinate die Anzahl der Bakterien (CFU, colony forming units), gemessen in einem Gramm Lunge, aufgetragen. Auf der Abszisse sind die unbehandelten bzw. behandelten Wildtyp-Mäuse bzw. Cftr-defizienten Mäuse einschließlich der verschiedenen Verabreichungsformen aufgeführt. Auf der Abszisse bedeuten die Zif-fern:
- 1:: Wildtyp-Mäuse, Bakterienzahlen in der Lunge 2 h nach der intranasalen Infektion, keine weitere Behandlung;
- 2:: Cftr-defiziente Mäuse, Bakterienzahlen in der Lunge 2 h nach der intranasalen Infektion, keine weitere Behandlung;
- 3:: Wildtyp-Mäuse, Bakterienzahlen in der Lunge 12 h nach Inhalation von 1 ml einer Amitriptylin-Lösung (4 mg/l) und 2 Stunden nach der intranasalen Infektion;
- 4:: Cftr-defiziente Mäuse, Bakterienzahlen in der Lunge 12 h nach Inhalation von 1 ml einer Amitriptylin-Lösung (4 mg/l) und 2 Stunden nach der intranasalen Infektion;
- 5:: Wildtyp-Mäuse, Bakterienzahlen in der Lunge 12 h nach intraperitonealer Injektion von 250 Mikrogramm Amitriptylin und 2 Stunden nach der intranasalen Infektion;
- 6:: Cftr-defiziente Mäuse, Bakterienzahlen in der Lunge 12 h nach intraperitonealer Injektion von 250 Mikrogramm Amitriptylin und 2 Stunden nach der intranasalen Infektion.

Der Figur 7 ist dabei deutlich zu entnehmen, dass die Verabreichung der erfindungsgemäßen Zusammensetzung zu einer weitgehenden Normalisierung der Infektionsanfälligkeit für Pseudomonas aeruginosa führt. Figur 7 verdeutlicht insbesondere die Eignung der erfindungsgemäßen Zusammensetzung zur Prophylaxe und/oder Therapie von Infektionen bzw. Infektionserkrankungen, die im Zusammenhang mit zystischer Fibrose auftreten. Bezüglich weiterer Vorteile wird ebenfalls auf das bereits unter 2.5.1 Gesagte verwiesen.

### 2.6 Untersuchung auf einen möglichen systemischen Effekt

Die Milz wurde den Versuchsmäusen 60 Minuten nach der letzten Inhalation entfernt. Danach wurde die Milz mechanisch zerkleinert. Die Splenozyten wurden zweimal in H/S gewaschen und in 250 mM Natriumacetat (pH 5.0), 1.3 mM EDTA und 1 % NP40 lysiert. Danach wurde die Aktivität der sauren Sphingomyelinase, wie zuvor beschrieben, bestimmt. Hierbei stellte sich heraus, dass die inhalative Verabreichung der Anti-depressiva keinerlei Auswirkung auf die Aktivität der sauren Sphingomyelinase in den Milzzellen (Splenozyten) der Versuchstiere hatte. Dies zeigt, dass im Falle einer inhalativen Verabreichung keine systemischen Effekte auftraten.

## Patentansprüche

1. Pharmazeutische Zusammensetzung in Form eines Inhalationsmittels zur Verwendung in der Prophylaxe und/oder symptomatischen Behandlung von zystischer Fibrose, insbesondere zur Verwendung in der Prophylaxe und/oder Behandlung von bei zystischer Fibrose auftretenden Infektionen und/oder Infektionserkrankungen, umfassend zumindest ein Antidepressivum aus der Gruppe Trimipramin, Chlorprothixen, Fluoxetin, Amlodipin, Sertralin und Kombinationen davon.

2. Pharmazeutische Zusammensetzung zur Verwendung nach Anspruch 1, **dadurch gekennzeichnet, dass** sie zumindest ein Dispersionsmittel und/oder zumindest ein pharmazeutisch verträgliches Trägermaterial umfasst.

3. Pharmazeutische Zusammensetzung zur Verwendung nach Anspruch 1 oder 2, **dadurch gekennzeichnet, dass** sie in Form als Aerosol, Spray oder wässrige Dispersion, insbesondere wässrige Lösung, vorliegt.

4. Pharmazeutische Zusammensetzung zur Verwendung nach einem der vorhergehenden Ansprüche, **dadurch gekennzeichnet, dass** das Antidepressivum in der Zusammensetzung in einer Dosis vorliegt, die 1/10 bis 1/1000 einer systemisch, insbesondere oral oder intravenös, verabreichten Dosis des Antidepressivums entspricht.

5. Pharmazeutische Zusammensetzung zur Verwendung nach einem der vorhergehenden Ansprüche, **dadurch gekennzeichnet, dass** die Zusammensetzung eine Menge des Antidepressivums zwischen 0,01 und 20 mg, insbesondere 0,1 und 10 mg, aufweist.

6. Pharmazeutische Zusammensetzung zur Verwendung nach einem der vorhergehenden Ansprüche, **dadurch gekennzeichnet, dass** die Infektionen bzw. Infektionserkrankungen zumindest von einem Erreger aus der Gruppe Pseudomonas aeruginosa, Pseudomonas species, Staphylococcus aureus, Burkholderia cepacia und Haemophilus influenzae verursacht sind.

7. Pharmazeutische Zusammensetzung zur Verwendung nach einem der vorhergehenden Ansprüche, **dadurch gekennzeichnet, dass** die Zusammensetzung Aerosol-Partikel mit einem Durchmesser zwischen 0,1 und 12 µm, insbesondere 1 und 12 µm, bevorzugt 2 und 12 µm, aufweist.

## Claims

1. A pharmaceutical composition in the form of an inhalant for use in prophylaxis and/or symptomatic treatment of cystic fibrosis, in particular for use in the prophylaxis and/or treatment of infections and/or infectious diseases occurring in cystic fibrosis, comprising at least one antidepressant from the group consisting of trimipramine, chlorprothixene, fluoxetine, amlodipine, sertraline, and combinations thereof.

2. The pharmaceutical composition for use according to claim 1, **characterized in that** the composition comprises at least one dispersant and/or at least one pharmaceutically compatible carrier material.

3. The pharmaceutical composition for use according to claim 1 or 2, **characterized in that** the composition is present in the form of an aerosol, spray or aqueous dispersion, in particular an aqueous solution.

4. The pharmaceutical composition for use according to any one of the preceding claims, **characterized in that** the antidepressant is present in the composition in a dose that corresponds to 1/10 to 1/1000 of a systemically, in particular orally or intravenously, administered dose of the antidepressant.

5. The pharmaceutical composition for use according to any one of the preceding claims, **characterized in that** the composition includes an amount of the antidepressant of between 0.01 and 20 mg, in particular 0.1 and 10 mg.

6. The pharmaceutical composition for use according to any one of the preceding claims, **characterized in that** the infections or infectious diseases are caused at least by one pathogen from the group consisting of *Pseudomonas aeruginosa, Pseudomo*nas species, *Staphylococcus aureus, Burkholderia cepacia* and *Haemophilus influenzae.*

7. The pharmaceutical composition for use according to any one of the preceding claims, **characterized in that** the composition includes aerosol particles with a diameter of between 0.1 and 12 µm, in particular 1 and 12 µm, preferably 2 and 12 µm.

## Revendications

1. Préparation pharmaceutique sous forme d'un agent d'inhalation, destinée à être utilisée dans la prophylaxie et/ou le traitement symptomatique de la fibrose kystique, en particulier destinée à être utilisée dans la prophylaxie et/ou le traitement d'infections et/ou de maladies infectieuses qui surviennent lors d'une fibrose kystique, comprenant au moins un antidépresseur du groupe formé par la trimipramine, le chloroprothixène, la fluoxétine, l'amlodipine, la sertraline et leurs combinaisons.

2. Préparation pharmaceutique destinée à une utilisation selon la revendication 1, **caractérisée en ce qu'**elle comprend au moins un dispersant et/ou au moins un matériau support pharmaceutiquement acceptable.

3. Préparation pharmaceutique destinée à une utilisation selon la revendication 1 ou 2, **caractérisée en ce qu'**elle se trouve sous forme d'aérosol, de spray ou de dispersion aqueuse, en particulier de solution aqueuse.

4. Préparation pharmaceutique destinée à une utilisation selon l'une quelconque des revendications précédentes, **caractérisée en ce que** l'antidépresseur se trouve, dans la préparation, en une dose qui correspond à 1/10 jusqu'à 1/1000 d'une dose d'antidépresseur administrée par voie systémique, en particulier par voie orale ou intraveineuse.

5. Préparation pharmaceutique destinée à une utilisation selon l'une quelconque des revendications précédentes, **caractérisée en ce que** la préparation présente une quantité d'antidépresseur entre 0,01 et 20 mg, en particulier entre 0,1 et 10 mg.

6. Préparation pharmaceutique destinée à une utilisation selon l'une quelconque des revendications précédentes, **caractérisée en ce que** les infections ou, selon le cas, les maladies infectieuses sont provoquées au moins par un agent pathogène du genre Pseudomonas aeruginosa, Pseudomonas species, Staphylococcus aureus, Burkholderia cepacia et Haemophilus influenzae.

7. Préparation pharmaceutique destinée à une utilisation selon l'une quelconque des revendications précédentes, **caractérisée en ce que** la composition présente des particules d'aérosol présentant un diamètre entre 0,1 et 12 µm, en particulier entre 1 et 12 µm, de préférence entre 2 et 12 µm.
